(19) Europäisches Patentamt
European Patent Office
Office européen des brevets

(11) EP 3 655 387 B1

(12) **EUROPEAN PATENT SPECIFICATION**

(45) Date of publication and mention of the grant of the patent:
**25.11.2020 Bulletin 2020/48**

(21) Application number: **19735348.5**

(22) Date of filing: **08.07.2019**

(51) Int Cl.:
***C07C 255/19*** *(2006.01)* ***C09D 133/26*** *(2006.01)*
***C08F 220/60*** *(2006.01)*

(86) International application number:
**PCT/EP2019/068206**

(87) International publication number:
**WO 2020/016037 (23.01.2020 Gazette 2020/04)**

(54) **CH-ACIDIC METHACRYLIC ESTERS FOR THE PREPARATION OF AQUEOUS POLYMER DISPERSIONS**

CH-ACIDE METHACRYLSÄUREESTER ZUR HERSTELLUNG VON WÄSSRIGEN POLYMERDISPERSIONEN

ESTER DE L'ACIDE MÉTHACRYLIQUE CH DESTINÉ À LA PRODUCTION DES DISPERSIONS POLYMÈRES AQUEUSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.07.2018 EP 18183897**

(43) Date of publication of application:
**27.05.2020 Bulletin 2020/22**

(73) Proprietor: **Evonik Operations GmbH**
**45128 Essen (DE)**

(72) Inventors:
- **TRESKOW, Marcel**
  **64293 Darmstadt (DE)**
- **CASPARI, Maik**
  **64665 Alsbach-Haehnlein (DE)**
- **SCHÜTZ, Thorben**
  **64665 Alsbach-Hähnlein (DE)**
- **KRILL, Steffen**
  **64367 Mühltal (DE)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(56) References cited:
**WO-A1-2009/146995** **US-A- 4 215 195**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 655 387 B1

## Description

The present invention relates to CH-acidic methacrylic esters and also copolymers obtainable from CH-acidic methacrylic esters. Furthermore, the present invention relates to coating compositions comprising these copolymers.

Room temperature crosslinking resins, the crosslinking mechanism of which is based on carbonyl group reactions, are known in the art and described for example in EP 0016518, DE 4237030, WO 2009/146995A1 or EP 2246403 as paint formulations or adhesive applications. Collectively, these resin systems generally have copolymers - in more modern implementations usually aqueous, VOC-free emulsion polymers formed from comonomers with lateral carbonyl function. Diacetone acrylamide (DAAM) or acetoacetoxyethyl methacrylate (AEEMA) are most commonly used for this purpose.

However, these two carbonyl-functional monomers have critical disadvantages: a relatively highly sterically hindered, and therefore poorly accessible, carbonyl function, while the ester group has significant sensitivity to hydrolysis. In practice, this leads to the coating compositions based on such comonomers having below-average storage stability. Furthermore, in the hydrolysis of such comonomers, carbon dioxide is formed which therefore leads to a considerable rise in pressure in the vessel, which as a result must have particularly high pressure resistance.

Added to this is the fact that monomers such as AAEMA and DAAM can only be prepared with a great deal of expenditure. The preparation of AAEMA requires diketene as raw material, which is toxic and has only limited storage stability. For the preparation of DAAM, the toxic acrylonitrile and the oleum, which can only be handled with difficulty, are required. Furthermore, this monomer is a solid, which generally makes the large-scale handleability thereof more difficult.

Added to this is the fact that copolymers based on acetoacetamides or the esters thereof form lightly coloured complexes with metal ions, even when the latter are only present in trace amounts. For this reason, such copolymers are unsuitable for a wide variety of clearcoat applications.

It was therefore an object of the present invention to overcome, or at least minimize, the stated disadvantages in the profile of properties of the copolymers based on AAEMA and DAAM and also the difficulties in the preparation thereof. In this case, it was essential to ensure that the CH-acidic monomer to be developed has sufficiently good water solubility and as a result can be subjected to an emulsion polymerization, even in the absence of organic solvents, and thus can provide virtually solvent-free aqueous dispersions.

Furthermore, along with this, it was also an object of the invention to provide a coating composition which has particularly long storability and shelf life. Furthermore, it was intended that the hardness of the coatings obtainable from the coating compositions be able to be varied over a wide range. It was especially intended that particularly hard, scratch-resistant coatings be able to be obtained.

A further object was that of providing copolymers, the use of which enabled coating compositions without volatile organic solvents to be obtained.

These objects were achieved by providing CH-acidic (meth)acrylates of the general formula (I):

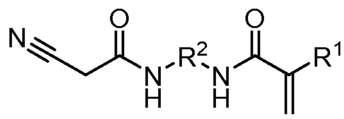

(I)

wherein $R^1$ is selected from a hydrogen atom and a methyl group and
$R^2$ is an optionally substituted alkylene group of the composition

$C_nH_m$

with

n = 2 - 4
m = 2 - 8

The CH-acidic (meth)acrylates of the general formula (I) have a CH-acidic functionality which is readily accessible for crosslinking, and are readily obtainable by the method described below. They can be used for further processing; generally this is polymerization in aqueous emulsion or suspension, without further work-up. The corresponding copolymers have a surprisingly high hydrolysis stability. Thus, products based on these copolymers are particularly storage-stable. In addition, even if a small amount of hydrolysis of such copolymers were to occur during storage, no gaseous compounds are formed in the process, leading to an undesired rise in pressure in the vessel.

Furthermore, the following advantages can be achieved by the use of inventive CH-acidic methacrylic esters of the formula (I):
The inventive CH-acidic methacrylic esters can be processed to give copolymers, coating compositions and coatings having a very low residual monomer content.

The hardness of the coatings which are obtainable from inventive coating compositions, which are in turn based on the copolymers or CH-acidic methacrylic esters, can be varied over a wide range. According to a preferred modification, according to the invention, especially particularly hard, scratch-resistant coatings can be obtained. The coatings obtainable from the coating compositions of the present invention show surprisingly high solvent resistance, which is especially apparent in experiments with methyl isobutyl ketone (MIBK), ammonia solutions or ethanol. Thus, the coatings obtained have an outstanding rating, especially in experiments according to the furniture test DIN 68861-1.

Coating compositions obtainable using the inventive CH-acidic methacrylic esters of the formula (I) generally do not require any volatile organic solvents. Moreover, the inventive coating compositions show particularly high storage stability, high shelf life and very good storability. In particular, there is virtually no aggregate formation.

The coatings obtainable from the inventive coating compositions also show high resistance to weathering, especially high UV resistance. Furthermore, the films obtainable from the coating compositions have, after a short time, low tackiness.

The inventive CH-acidic methacrylic esters of the formula (I), copolymers and coating compositions can be prepared on a large scale in a cost-effective manner. The inventive coating compositions are environmentally friendly and can be processed and prepared safely and without a great deal of expenditure. Here, the inventive coating compositions have outstanding shear stability.

According to the invention, the radical $R^1$ in the general formula (I) can be a hydrogen atom or a methyl group, wherein $R^1$ is preferably a methyl group.

According to the invention, the radical $R^2$ is an optionally substituted alkylene group of the composition

$$C_nH_m$$

with

$n = 2 - 4$
$m = 2 - 8$

The radical $R^2$ may be branched or unbranched. Furthermore, $R^2$ may have one or more substituents, especially halogen atoms or hydroxy groups. The inventors determined, surprisingly, that the number of carbon atoms in the radical $R^2$ has a defining influence on the solubility of the CH-acidic methacrylic esters of the formula (I) in water. Thus, corresponding compounds having 2-4 carbon atoms in the radical $R^2$ have outstanding water-solubility. Consequently, these compounds can be used even in the absence of an organic solvent in an emulsion polymerization for the preparation of aqueous dispersions. This makes it possible to prepare virtually solvent-free aqueous dispersions, which are advantageous for toxicological reasons.

On the contrary, comparable CH-acidic methacrylic esters with more than 6 carbon atoms in the radical $R^2$ are virtually insoluble in water. As a result, emulsion polymerization of such compounds generally requires the addition of a water-soluble organic solvent, for example ethanol. The preparation of solvent-free aqueous dispersions is therefore not possible.

Particularly preferred alkylene groups $R^2$ can for example be one of the following groups: 1,2-ethylene group, 1,3-propylene group, 1,2-propylene group, 1,4-butylene group, 1,3-(2-hydroxy)propylene group, 1,3-(2,2-dimethyl)propylene group, wherein it has proved particularly advantageous if the alkylene group $R^2$ is a 1,2-ethylene group or a 1,4-butylene group.

The inventors determined, surprisingly, that the inventive (meth)acrylates of the general formula (I) are obtainable by a method in which an ester of cyanoacetic acid (A) is reacted with a diamine (B) to give the corresponding cyanoacetamide (IP), and the cyanoacetamide (IP) is reacted in a subsequent process step with a (meth)acrylic derivative (C) to give the inventive (meth)acrylate of the general formula (I). The inventive compound of the general formula (I) can thereby be isolated in a particularly high product yield and a high degree of purity.

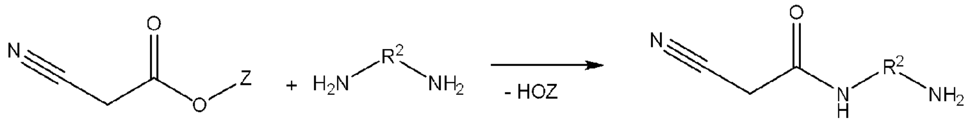


A B IP

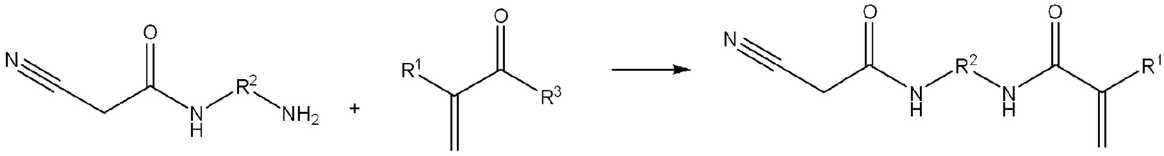

IP C I

In a preferred embodiment, the method for preparing the inventive compound of the general formula (I) has the following process steps:

a) reacting an ester of cyanoacetic acid (A) with a diamine (B) which is present in excess, with a cyanoacetamide (IP) being formed;
b) removing the unreacted diamine (B),
c) reacting the cyanoacetamide (IP) from the method step b) either
c1) with a (meth)acrylic ester (C1), or
c2) with a (meth)acrylic anhydride (C2), or
c3) with a (meth)acryloyl halide (C3), and
d) optionally isolating the compound of the general formula (I) using extraction or crystallization.

As esters of cyanoacetic acid (A), use is preferably made of methyl and ethyl esters (Z = $CH_3$, $C_2H_5$) of cyanoacetic acid, because these give the desired cyanoacetamide (IP) in particularly high yields.
In addition, it has proved advantageous in the preparation of the cyanoacetamide (IP) to work with an excess of diamine (B), in order to suppress formation of undesired by-products. The diamine (B) generally has a lower boiling point than the cyanoacetamide (IP) and can therefore be removed by distillation in the process step b) with little complexity. Surprisingly, the formation of undesired by-products can be effectively suppressed even with small excesses of diamine (B), for example with a molar ratio of B to A of at least 1.001 : 1. At a molar ratio of B to A of at least 10 : 1, particularly preferably of at least 4 : 1, the cyanoacetamide (IP) is generally obtained, which even as crude product, after the removal of the excess diamine (B), is isolated with a degree of purity of more than 99%. The separation of diamine (B) can in this case be carried out by extraction or crystallization but especially by distillation, preferably under reduced pressure.
Suitable diamines (B) for the preparation of the compound of the general formula (I) can be selected substantially unrestrictedly from the group of the aliphatic, linear or branched or cyclic substituted and unsubstituted diamines. Particularly preferred diamines are selected from the group comprising 1,2-ethylenediamine, 1,3-diaminopropane, 1,4-diaminobutane, 1,2-diaminopropane and 1,3-diamino-2-hydroxypropane**.**
In a particularly preferred embodiment, the cyanoacetamide (IP) is reacted to give the compound of the general formula (I) using a (meth)acrylic anhydride (C2) or a (meth)acryloyl halide (C3). Here, the use of methacrylic anhydride and acrylic anhydride has proved to be particularly advantageous.
In the present patent application, the notation *"(meth)acrylate"* here means both methacrylate, for example methyl methacrylate, ethyl methacrylate, etc., and acrylate, for example methyl acrylate, ethyl acrylate, etc., and mixtures of the two.
Particularly high yields of the compound of the general formula (I) can especially be achieved by the molar ratio of C to IP in the process step c) being in the range from 0.2 : 1 to 5 : 1, preferably in the range from 0.8 : 1 to 2 : 1, particularly preferably in the range from 0.9 : 1 to 1.5 : 1.
The reaction in the process step a) is typically carried out at temperatures between 0°C and 120°C, preferably between 10°C and 40°C during the metering, and at temperatures up to 100°C in the post-reaction phase and preparation for work-up.
In the process step c1) it has proved to be advantageous to carry out the reaction at a temperature in the range from 60°C to 140°C, preferably at 100°C to 120°C. In the process step c2), on the other hand, the reaction is preferably carried out at a reaction temperature of approximately 0°C to 40°C; optionally in the post-reaction up to 100°C. In order to avoid the formation of undesired by-products, the reaction temperature is kept as low as possible.
The reaction in the process step c) may occur in the presence of a catalyst. Metal compounds and/or amines catalyse the reactions of (meth)acrylic anhydride with hydroxy or amine groups multiple times. Metal compounds and

amines are known in the art and set out, for example, in Ullmann's Encyclopedia of Industrial Chemistry (6th edition), Wiley-VCH publishing, Weinheim 2003 or Römpp Chemielexikon, 2nd edition, on CD-ROM. In particular, salts such as, e.g., halides, hydroxides or oxides of alkali metals, such as LiOH, KOH or zirconium compounds belong to the metal compounds. For example, ammonia, triethylamine, tributylamine, and others, belong to the amines.

Use is preferably made of polymerization inhibitors in the reaction. These compounds, for example hydroquinones, hydroquinone ethers, such as hydroquinone monomethyl ether or di-tert-butylcatechol, phenothiazine, 4-hydroxy-2,2,6,6-tetramethylpiperidin-1-oxyl, methylene blue or sterically hindered phenols, for example 2,4-dimethyl-6-tert-butylphenol, are widely known in the art. These compounds can be used individually or in the form of mixtures and are generally commercially available. Further details may be found in the relevant specialist literature, in particular Römpp-Lexikon Chemie; editors: J. Falbe, M. Regitz; Stuttgart, New York; 10th edition (1996); keyword "Antioxidantien" and the literature references cited there.

Particular preference is given to using phenols as polymerization inhibitor. Particularly surprising advantages can be achieved by using mixtures which comprise hydroquinone monomethyl ether and/or 2,4-dimethyl-6-tert-butylphenol. The molar ratio of hydroquinone monomethyl ether to 2,4-dimethyl-6-tert-butylphenol is particularly preferably in the range from 2 : 1 to 1 : 2. Based on the weight of the total reaction mixture, the proportion of inhibitors, either individually or as a mixture, can generally be 0.01 to 0.5% (wt/wt).

These polymerization inhibitors may be added to the reaction mixture prior to, or at the start of, the reaction. Moreover, some of the added polymerization inhibitors may also be added during the reaction.

In a preferred embodiment, the reaction can be effected in the presence of oxygen, especially atmospheric oxygen. Moreover, the reaction can furthermore occur under an oxygen-depleted atmosphere, especially under nitrogen.

The reaction duration in process steps a) and c) is typically 15 min to 10 hours, preferably 1 hour to 5 hours. The reaction may take place at increased pressure or reduced pressure. According to a particularly expedient modification of the present invention, the reactions can be carried out at a pressure in the range from 200 mbar to 2000 mbar, particularly preferably in the range from 500 mbar to 1300 mbar.

The cyanoacetamide (IP) can be further used without aqueous work-up. The cyanoacetamide (IP) from the process step b) is preferably taken up hot in a solvent, since otherwise it would solidify to a glass-like mass, and it thus reacts considerably better. Suitable solvents for this purpose are water, methyl-tert-butyl ether (MTBE), tetrahydrofuran (THF), acetonitrile, dioxane and alcohols. The selection is obvious for those skilled in the art, based on the respective purpose of the reaction.

In the process step c2), a solvent is preferably employed such as water, MTBE, THF, acetonitrile, dioxane and alcohols or mixtures thereof. The (meth)acrylic acid or salts thereof contained in the reaction mixture can remain in the crude product obtained, without this having a disadvantageous influence on the copolymers obtained therefrom. Depending on the intended use of the copolymers, however, the (meth)acrylic acid obtained may be removed from the reaction mixture by extraction methods or distillation.

The inventive compound of the general formula (I) can be used further without work-up. If required, it can also be dried under reduced pressure, be recrystallized by addition of a polar solvent, or be extracted by addition of an immiscible solvent.

According to one configuration of the inventive method, the product mixture obtained can be purified by filtration methods. These methods are known from the prior art (W. Gösele, Chr. Alt in Ullmann's Encyclopedia of Industrial Chemistry, (6th edition), Wiley-VCH publishing, Weinheim 2003, volume 13, pages 731 and 746), with customary filtration aids, for example bleaching earth and aluminium silicates (perlite), being able to be used. For example, inter alia, use may be made of continuously operable filters for precoat filtration.

Copolymers based on the CH-acidic methacrylic esters

A further aspect of the present invention relates to copolymers based on the CH-acidic methacrylic esters. Corresponding copolymers are obtainable by polymerization of a monomer mixture, which typically comprises at least 0.5 wt%, preferably at least 1 wt% of a CH-acidic (meth)acrylate of the general formula (I), based on the weight of the monomer mixture.

As well as at least one (meth)acrylate monomer according to formula (I), the monomer mixture comprises at least one further monomer, which is copolymerizable with the (meth)acrylate monomer according to formula (I). These copolymerizable monomers include monomers with an acid group, monomers comprising ester groups which differ from the (meth)acrylate monomer according to formula (I), and styrene monomers.

Monomers containing acid groups are compounds which can be preferably radically copolymerized with the abovementioned (meth)acrylate monomers according to formula (I). These include for example monomers with a sulfonic acid group, for example vinylsulfonic acid; monomers with a phosphonic acid group, for example vinylphosphonic acid and unsaturated carboxylic acids, for example methacrylic acid, acrylic acid, fumaric acid and maleic acid. Methacrylic acid and acrylic acid are particularly preferred. The monomers containing acid groups may be used individually or as a

mixture of two, three or more monomers containing acid groups.

The preferred monomers comprising ester groups include especially (meth)acrylates which differ from the monomers according to formula (I), fumarates, maleates and/or vinyl acetate. The expression (meth)acrylates encompasses methacrylates and acrylates and also mixtures thereof. These monomers are well-known.

They include, especially, (meth)acrylates having 1 to 6 carbon atoms in the alkyl radical and which are derived from saturated alcohols, such as methyl (meth)acrylate, ethyl (meth)acrylate, n-propyl (meth)acrylate, isopropyl (meth)acrylate, n-butyl (meth)acrylate, tert-butyl (meth)acrylate and pentyl (meth)acrylate, hexyl (meth)acrylate; cycloalkyl (meth)acrylates, such as cyclopentyl (meth)acrylate, cyclohexyl (meth)acrylate; and (meth)acrylates which are derived from unsaturated alcohols, such as 2-propynyl (meth)acrylate, allyl (meth)acrylate and vinyl (meth)acrylate.

For the preparation of inventive copolymers, particular preference is given to using mixtures which comprise methacrylates and acrylates. Thus, use may especially be made of mixtures of methyl methacrylate and acrylates having 2 to 6 carbon atoms, such as ethyl acrylate, butyl acrylate and hexyl acrylate.

Moreover, the comonomers include, for example, (meth)acrylates having at least 7 carbon atoms in the alkyl radical and which are derived from saturated alcohols, for example 2-ethylhexyl (meth)acrylate, heptyl (meth)acrylate, 2-tert-butylheptyl (meth)acrylate, octyl (meth)acrylate, 3-isopropylheptyl (meth)acrylate, nonyl (meth)acrylate, decyl (meth)acrylate, undecyl (meth)acrylate, 5-methylundecyl (meth)acrylate, dodecyl (meth)acrylate, 2-methyldodecyl (meth)acrylate, tridecyl (meth)acrylate, 5-methyltridecyl (meth)acrylate, tetradecyl (meth)acrylate, pentadecyl (meth)acrylate, hexadecyl (meth)acrylate, 2-methylhexadecyl (meth)acrylate, heptadecyl (meth)acrylate, 5-isopropylheptadecyl (meth)acrylate, 4-tert-butyloctadecyl (meth)acrylate, 5-ethyloctadecyl (meth)acrylate, 3-isopropyloctadecyl (meth)acrylate, octadecyl (meth)acrylate, nonadecyl (meth)acrylate, eicosyl (meth)acrylate, cetyleicosyl (meth)acrylate, stearyleicosyl (meth)acrylate, docosyl (meth)acrylate, and/or eicosyltetratriacontyl (meth)acrylate; cycloalkyl (meth)acrylates such as 3-vinylcyclohexyl (meth)acrylate, isobornyl (meth)acrylate, cycloalkyl (meth)acrylates, such as 2,4,5-tri-tert-butyl-3-vinylcyclohexyl (meth)acrylate, 2,3,4,5-tetra-tert-butylcyclohexyl (meth)acrylate; heterocyclic (meth)acrylates, such as 2-(1-imidazolyl)ethyl (meth)acrylate, 2-(4-morpholinyl)ethyl (meth)acrylate and 1-(2-methacryloyloxyethyl)-2-pyrrolidone; nitriles of (meth)acrylic acid and other nitrogenous methacrylates, such as N-(methacryloyloxyethyl)diisobutyl ketimine, N-(methacryloyloxyethyl)dihexadecyl ketimine, methacryloylamidoacetonitrile, 2-methacryloyloxyethylmethyl cyanamide, cyanomethyl methacrylate; aryl (meth)acrylates, such as benzyl (meth)acrylate or phenyl (meth)acrylate, wherein the aryl radicals can in each case be unsubstituted or up to quadruply substituted; (meth)acrylates which have two or more (meth)acryloyl groups, glycol di(meth)acrylates, such as ethylene glycol di(meth)acrylate, diethylene glycol di(meth)acrylate, triethylene glycol di(meth)acrylate, tetra- and polyethylene glycol di(meth)acrylate, 1,3-butanediol (meth)acrylate, 1,4-butanediol (meth)acrylate, 1,6-hexanediol di(meth)acrylate, glycerol di(meth)acrylate; dimethacrylates of ethoxylated bisphenol A; (meth)acrylates having three or more double bonds, e.g. glycerol tri(meth)acrylate, trimethylolpropane tri(meth)acrylate, pentaerythritol tetra(meth)acrylate and dipentaerythritol penta(meth)acrylate; (meth)acrylates, functional methacrylates with ether or amine groups in the side chain.

Moreover, the monomers comprising ester groups include vinyl esters, such as vinyl acetate; maleic acid derivatives, for example maleic anhydride, esters of maleic acid, for example dimethyl maleate, methylmaleic anhydride; and fumaric acid derivatives, such as dimethyl fumarate.

A further preferred group of comonomers are styrene monomers, for example styrene, substituted styrenes having an alkyl substituent in the side chain, for example α-methylstyrene and α-ethylstyrene, substituted styrenes having an alkyl substituent on the ring, such as vinyltoluene and p-methylstyrene, halogenated styrenes, for example monochlorostyrenes, dichlorostyrenes, tribromostyrenes and tetrabromostyrenes.

As well as the abovementioned monomers, inventive polymers which are obtained by the polymerization of monomer mixtures may contain further monomers. These include, for example, heterocyclic vinyl compounds, such as 2-vinylpyridine, 3-vinylpyridine, 2-methyl-5-vinylpyridine, 3-ethyl-4-vinylpyridine, 2,3-dimethyl-5-vinylpyridine, vinylpyrimidine, vinylpiperidine, 9-vinylcarbazole, 3-vinylcarbazole, 4-vinylcarbazole, 1-vinylimidazole, 2-methyl-1-vinylimidazole, N-vinylpyrrolidone, 2-vinylpyrrolidone, N-vinylpyrrolidine, 3-vinylpyrrolidine, N-vinylcaprolactam, N-vinylbutyrolactam, vinyloxolane, vinylfuran, vinylthiophene, vinylthiolan, vinylthiazoles and hydrogenated vinylthiazoles, vinyloxazoles and hydrogenated vinyloxazoles; maleimide, methylmaleimide; vinyl-and isoprenyl ether; and vinyl halides, for example vinyl chloride, vinyl fluoride, vinylidene chloride and vinylidene fluoride.

Monomer mixtures used with preference according to the invention comprise:

0.1 to 90 wt%, preferably 0.5 to 30 wt%, of (meth)acrylate monomer according to formula (I);
10 to 95 wt%, preferably 40 to 90 wt%, of monomers with ester groups;
0 to 20 wt%, preferably 1 to 8 wt%, especially 1 - 3 wt%, of monomer with an acid group, and
0 to 70 wt%, preferably 0 to 50 wt%, especially 0 - 30 wt% of styrene monomers, with the figures in each case relating to the total weight of the monomers.

Monomer mixtures used according to the invention having a high proportion of (meth)acrylate monomer according to formula (I) generally lead to polymers or coating compositions which make it possible to obtain particularly weathering-stable, solvent-resistant and hard coatings.

These monomer mixtures preferably comprise

10 to 90 wt%, preferably 15 to 40 wt%, of (meth)acrylate monomer according to formula (I);
10 to 90 wt%, preferably 40 to 85 wt%, of monomers with ester groups;
0 to 10 wt%, preferably 1 to 8 wt%, of monomer with an acid group, and
0 to 50 wt%, preferably 0 to 30 wt% of styrene monomers, with the figures in each case relating to the total weight of the monomers in the monomer mixture.

The inventive CH-acidic (meth)acrylic esters of the formula (I) especially serve for the preparation or the modification of copolymers. The polymerization may be carried out in any known manner. This includes, especially, radical, cationic or anionic polymerization, with variants of these polymerization methods, for example ATRP (=Atom Transfer Radical Polymerization), NMP (Nitroxide Mediated Polymerization) or RAFT (=Reversible Addition Fragmentation Chain Transfer), also being able to be used.

The abovementioned monomers or CH-acidic methacrylic esters of the formula (I) may for example be converted by solution polymerizations, bulk polymerizations or emulsion polymerizations, with surprising advantages being able to be achieved by radical emulsion polymerization.

A further aspect of the present invention relates to an aqueous dispersion which contains the inventive copolymer. The content of the copolymer in the dispersion is generally 0.1 to 90 wt%, preferably 20 to 80 wt%, particularly preferably 30 to 60 wt%, based on the weight of the aqueous dispersion. Such aqueous dispersions can especially be prepared by emulsion polymerization.

Methods of emulsion polymerization are *inter alia* described in Ullmann's Encyclopedia of Industrial Chemistry, Fifth Edition. In general, for this purpose an aqueous phase is prepared which, as well as water, can comprise customary additives, especially emulsifiers and protective colloids for the stabilization of the emulsion.

Monomers are subsequently added to this aqueous phase and polymerized in the aqueous phase. In the preparation of homogeneous polymer particles, in this case a CH-acidic methacrylic ester can be added continuously or batchwise over a period of time.

The emulsion polymerization may for example be implemented as mini- or microemulsion, which are described in more detail in Chemistry and Technology of Emulsion Polymerisation, A.M. van Herk (editor), Blackwell Publishing, Oxford 2005 and J. O'Donnell, E.W. Kaler, Macromolecular Rapid Communications 2007, 28(14), 1445-1454. A miniemulsion is usually characterized by the use of costabilizers or swelling agents, with long-chain alkanes or alkanols often being used. The droplet size in miniemulsions is preferably in the range from 0.05 μm to 20 μm. The droplet size in microemulsions is preferably in the range of below 1 μm, as a result of which particles smaller than a size of 50 nm can be obtained. In microemulsions, additional surfactants are often used, for example hexanol or similar compounds.

The dispersing of the monomer-containing phase in the aqueous phase may take place using known means. These include especially mechanical methods and also the use of ultrasound.

In the preparation of homogeneous emulsion polymers, use may preferably be made of monomer mixtures which comprise 0.5 to 30 wt% of a CH-acidic methacrylic ester of the formula (I).

In the preparation of core-shell polymers, the composition of the monomer mixture may be modified in a stepwise manner, with, prior to the modification of the composition, the polymerization preferably having been carried out up to a conversion of at least 80 wt%, particularly preferably at least 95 wt%, in each case based on the total weight of the monomer mixture used. The progress of the polymerization reaction in each process step can be monitored in a known manner, for example by gravimetric means or by means of gas chromatography.

The emulsion polymerization is carried out preferably at a temperature in the range from 0°C to 120°C, particularly preferably in the range from 30°C to 100°C. In this case, polymerization temperatures in the range from greater than 60°C to less than 90°C, expediently in the range from greater than 70°C to less than 85°C, preferably in the range from greater than 75°C to less than 85°C, have proved to be particularly favourable.

The initiation of the polymerization is effected with the initiators that are commonly used for emulsion polymerization. Suitable organic initiators are, for example, hydroperoxides such as tert-butyl hydroperoxide or cumene hydroperoxide. Suitable inorganic initiators are hydrogen peroxide and the alkali metal and ammonium salts of peroxodisulfuric acid, especially ammonium, sodium and potassium peroxodisulfate. Suitable redox initiator systems are for example combinations of tertiary amines with peroxides or sodium disulfite and alkali metal and ammonium salts of peroxodisulfuric acid, especially sodium and potassium peroxodisulfate. Further details may be found in the specialist literature, especially H. Rauch-Puntigam, Th. Völker, "Acryl- und Methacrylverbindungen" [acrylic and methacrylic compounds], Springer, Heidelberg, 1967 or Kirk-Othmer, Encyclopedia of Chemical Technology, vol. 1, pages 386ff, J. Wiley, New York, 1978. In the context of the present invention, the use of organic and/or inorganic initiators is particularly preferred.

Said initiators can be used individually or as a mixture. They are preferably used in an amount of 0.05 to 3.0 wt% based on the total weight of the monomers in the particular stage. It is also possible to preferably carry out the polymerization with a mix of different polymerization initiators with different half-life values, in order to keep the supply of radicals constant over the course of the polymerization and also at different polymerization temperatures.

The mixture is preferably stabilized by means of emulsifiers and/or protective colloids. The emulsion is preferably stabilized by emulsifiers, in order to obtain low dispersion viscosity. The total amount of emulsifier is preferably from 0.1 to 15 wt%, especially 1 to 10 wt%, and particularly preferably 2 to 5 wt%, based on the total weight of the monomers used. According to a further aspect of the present invention, a portion of the emulsifiers can be added during the polymerization.

Particularly suitable emulsifiers are anionic or nonionic emulsifiers or mixtures thereof, especially

- alkyl sulfates, preferably those having 8 to 18 carbon atoms in the alkyl radical, alkyl and alkylaryl ether sulfates having 8 to 18 carbon atoms in the alkyl radical and 1 to 50 ethylene oxide units;
- sulfonates, preferably alkylsulfonates having 8 to 18 carbon atoms in the alkyl radical, alkylarylsulfonates having 8 to 18 carbon atoms in the alkyl radical, esters of sulfosuccinic acid with monohydric alcohols or alkylphenols having 4 to 15 carbon atoms in the alkyl radical; these alcohols or alkylphenols may also optionally be ethoxylated with 1 to 40 ethylene oxide units;
- phosphoric partial esters and the alkali metal and ammonium salts thereof, preferably alkyl and alkylaryl phosphates having 8 to 20 carbon atoms in the alkyl or alkylaryl radical and 1 to 5 ethylene oxide units;
- alkyl polyglycol ethers, preferably having 8 to 20 carbon atoms in the alkyl radical and 8 to 40 ethylene oxide units;
- alkylaryl polyglycol ethers, preferably having 8 to 20 carbon atoms in the alkyl or alkylaryl radical and 8 to 40 ethylene oxide units;
- ethylene oxide/propylene oxide copolymers, preferably block copolymers, favourably having 8 to 40 ethylene oxide and/or propylene oxide units.

The particularly preferred anionic emulsifiers include especially fatty alcohol ether sulfates, diisooctylsulfosuccinate, lauryl sulfate, C15 paraffin sulfonate, with these compounds generally being able to be used as alkali metal salt, especially as sodium salt. These compounds are commercially available especially under the trade names Disponil® FES 32, Aerosol® OT 75, Texapon® K1296 und Statexan® K1 from the companies Cognis GmbH, Cytec Industries, Inc. and Bayer AG.

Expediently, nonionic emulsifiers are *inter alia* tert-octylphenol ethoxylate with 30 ethylene oxide units and fatty alcohol polyethylene glycol ethers which preferably have 8 to 20 carbon atoms in the alkyl radical and 8 to 40 ethylene oxide units. These emulsifiers are commercially available under the trade names Triton® X 305 (Fluka), Tergitol® 15-S-7 (Sigma-Aldrich Co.), Marlipal® 1618/25 (Sasol Germany) and Marlipal® O 13/400 (Sasol Germany).

Use may preferably be made of mixtures of anionic emulsifier and nonionic emulsifier. Expediently, the weight ratio of anionic emulsifier to nonionic emulsifier may be in the range from 20 : 1 to 1 : 20, preferably from 2 : 1 to 1 : 10 and more preferably 1 : 1 to 1 : 5. In this case, mixtures containing a sulfate, especially a fatty alcohol ether sulfate, a lauryl sulfate, or a sulfonate, especially a diisooctyl sulfosuccinate or a paraffin sulfonate as anionic emulsifier and an alkylphenol ethoxylate or a fatty alcohol polyethylene glycol ether, which each preferably have 8 to 20 carbon atoms in the alkyl radical and 8 to 40 ethylene oxide units, have proved particularly successful as nonionic emulsifiers.

Optionally, the emulsifiers may also be used in a mixture with protective colloids. Suitable protective colloids include partly hydrolysed polyvinylacetates, polyvinylpyrrolidones, carboxylmethyl, methyl, hydroxyethyl and hydroxypropyl cellulose, starches, proteins, poly(meth)acrylic acid, poly(meth)acrylamide, polyvinylsulfonic acids, melamine formaldehydesulfonates, naphthalene formaldehydesulfonates, styrene-maleic acid and vinyl ether-maleic acid copolymers. If protective colloids are used, they are preferably used in an amount of 0.01 1.0 wt%, based on the total amount of the monomers. The protective colloids can be initially charged or metered in before the start of the polymerization. The initiator can be initially charged or metered in. In addition, it is also possible to initially charge a portion of the initiator and to meter in the remainder.

Preferably, the polymerization is started by heating the mixture to the polymerization temperature and metering in the initiator, preferably in aqueous solution. The metered additions of emulsifier and monomers can be conducted separately or as a mixture. In the case of metered addition of mixtures of emulsifier and monomer, the procedure is to premix emulsifier and monomer in a mixer connected upstream of the polymerization reactor. Preferably, the remainders of emulsifier and monomer which have not been initially charged are metered in separately after the polymerization has started. Preferably, the metered addition can be commenced 15 to 35 minutes after the polymerization has started.

Preferred emulsion polymers having a high proportion of insoluble polymers can be obtained in the above-described way, with the reaction parameters for obtaining a high molecular weight being known. Thus, the use of molecular weight regulators can thereby in particular be dispensed with.

The adjustment of the particle radii can be influenced, *inter alia,* by the proportion of emulsifiers. The higher

this proportion, especially at the start of the polymerization, the smaller the particles obtained.

The polymers obtainable according to the above-described method, especially the emulsion polymers preferably obtainable, are a further subject of the present invention.

According to a preferred modification of the present invention, an emulsion polymer may have a swelling of at least 1000%, particularly preferably at least 1400%, and most particularly preferably at least 1600% in tetrahydrofuran (THF) at 20°C. The upper limit value of the swelling is not critical *per se,* with the swelling being preferably at most 5000%, particularly preferably at most 3000% and most particularly preferably at most 2500%. In order to determine the swelling, a sample of the emulsion polymer dried with exclusion of oxygen is stored in a 200-fold amount of THF at 20°C for 4 hours. The sample swells as a result. The sample swollen in this way is separated from the remaining solvent. The solvent is subsequently removed from the sample. For example, a large proportion of the solvent can be evaporated at room temperature (20°C). Solvent residues can be removed in a drying cabinet (140°C), with this generally being achieved within 1 hour. The swelling is given by the weight of the solvent taken up by the sample and the weight of the dry sample. Moreover, the soluble fraction of the emulsion polymer is given by the difference in the weight of the sample before the swelling experiment and the weight of the dried sample after the swelling experiment.

The particle radius of the emulsion polymers can be within a wide range. Thus, use may especially be made of emulsion polymers with a particle radius in the range from 1 to 100 nm, preferably 5 to 59 nm. According to a further aspect of the present invention, the radius of the particles is preferably in the range from 60 nm to 500 nm, particularly preferably 70 to 150 nm and most particularly preferably 75 to 100 nm. The radius of the particles can be determined by PCS (photon correlation spectroscopy), with the data given relating to the d50 value (50% of the particles are smaller, 50% are bigger). For this purpose, for example, use may be made of a Beckman Coulter N5 Submicron Particle Size Analyzer.

Surprising advantages are exhibited especially by emulsion polymers with a high swelling factor. Preferred emulsion polymers have a swelling factor of at least 2, especially at least 4, particularly preferably at least 6 and most particularly preferably at least 8. In order to determine the swelling factor, firstly the particle radius of the emulsion polymers is measured in water with the above-described method. Subsequently, the emulsion polymers are swollen in a solvent/water mixture (THF/water = 90 : 10) and the particle sizes (microgels) are quantified by means of measuring with the Coulter Nanosizer N5 (rsolv.). Usually, a corresponding amount of tetrahydrofuran (THF) is added for this purpose to the dispersion, in order to adjust the volume ratio of THF/water in the dispersion to 90 : 10. The measurement is carried out at 20°C, with the dispersion being swollen for 5 minutes after the addition of the solvent (THF). The quotient of the particle volumes calculated from the particle radii obtained (rsolv. and rwater) is defined as the swelling factor (SF):

$$\mathrm{SF} = \frac{r^3_{\mathrm{solv}}}{r^3_{\mathrm{water}}}$$

Emulsion polymers with high swelling factors are those having low pre-crosslinking. Pre-crosslinking of the emulsion polymer arises by polymerization in the presence of monomers comprising more than one polymerizable double bond (for example a further methacrylate group). Such monomers are sometimes referred to as crosslinkers, since they make it possible to connect two polymer chains to one another. Formally, this corresponds to regular crosslinking in the coating of articles, however the timing of the crosslinking, prior to application, is undesired. Emulsion polymers which were prepared from CH-acidic methacrylic esters with a low proportion of crosslinkers accordingly exhibit a high swelling factor.

The glass transition temperature of the inventive polymer is preferably in the range from -30°C to 70°C, particularly preferably in the range from -20 to 40°C and most particularly preferably in the range from 0 to 25°C. The glass transition temperature can be influenced by the type and proportion of the monomers used to prepare the polymer. The glass transition temperature Tg of the polymer can be determined here in a known manner by means of differential scanning calorimetry (DSC). In addition, the glass transition temperature Tg can also be calculated approximately in advance by means of the Fox equation. According to Fox T. G., Bull. Am. Physics Soc. 1, 3, page 123 (1956):

$$\frac{1}{Tg} = \frac{x_1}{Tg_1} + \frac{x_2}{Tg_2} + \ldots + \frac{x_n}{Tg_n}$$

where $x_n$ is the mass fraction (wt%/100) of the monomer n and $Tg_n$ is the glass transition temperature in kelvin of the homopolymer of the monomer n. Further helpful pointers can be found by the person skilled in the art in Polymer Handbook 2nd Edition, J. Wiley & Sons, New York (1975), which gives Tg values for the most common homopolymers. In this context, the polymer may have one or more different glass transition temperatures. These figures therefore apply

to one segment obtainable by polymerization of an inventive CH-acidic methacrylic ester.

The architecture of the copolymer is not critical for many applications and properties. Accordingly, the copolymers, especially the emulsion polymers, can be random copolymers, gradient copolymers, block copolymers and/or graft copolymers. Block copolymers or gradient copolymers can for example be obtained by changing the monomer composition in a discontinuous manner while the chain is growing. According to a preferred aspect of the present invention, the emulsion polymer is a random copolymer in which the monomer composition is substantially constant over the polymerization. However, since the monomers can have different copolymerization parameters, the precise composition may fluctuate over the polymer chain of the polymer.

The polymer may be a homogeneous polymer, which for example in an aqueous dispersion forms particles with a consistent composition. In this case, the polymer, which is preferably an emulsion polymer, may consist of one or more segments which are obtainable by polymerization of the inventive CH-acidic methacrylic esters of the formula (I).

According to a further embodiment, the emulsion polymer may be a core-shell polymer, which may have one, two, three or more shells. In this case, the segment which is obtainable by polymerization of the inventive CH-acidic methacrylic esters of the formula (I) preferably forms the outermost shell of the core-shell polymer. The shell can be connected to the core or to the inner shells via covalent bonds. Furthermore, the shall can also be polymerized onto the core or an inner shell. In this embodiment, the segment which is obtainable by polymerization of the inventive CH-acidic methacrylic esters of the formula (I) can be separated and isolated from the core in many ways by suitable solvents.

Preferably, the weight ratio of segment which is obtainable by polymerization of the inventive CH-acidic methacrylic esters of the formula (I) to core is in the range from 2 : 1 to 1 : 6, particularly preferably 1 : 1 to 1 : 3.

The core may preferably be formed from polymers which comprise 50 to 100 wt%, preferably 60 to 90 wt% of units derived from (meth)acrylates. In this context, preference is given to esters of (meth)acrylic acid, the alcohol radical of which preferably comprises 1 to 30 carbon atoms, particularly preferably 1 to 20 carbon atoms and most particularly preferably 1 to 10 carbon atoms. These include, especially, (meth)acrylates which are derived from saturated alcohols, such as methyl (meth)acrylate, ethyl (meth)acrylate, n-propyl (meth)acrylate, isopropyl (meth)acrylate, n-butyl (meth)acrylate, tert-butyl (meth)acrylate and pentyl (meth)acrylate, hexyl (meth)acrylate.

According to a particular configuration of the present invention, a mixture comprising methacrylates and acrylates can be used for the preparation of the core. Thus, use may especially be made of mixtures of methyl methacrylate and acrylates having 2 to 6 carbon atoms, such as ethyl acrylate, butyl acrylate and hexyl acrylate.

Moreover, the polymers of the core may comprise the above-described comonomers. According to a preferred modification, the core can be crosslinked. This crosslinking can be achieved by the use of monomers with two, three or more radically polymerizable double bonds.

According to a particular aspect, the core may preferably have a glass transition temperature in the range from -30°C to 200°C, particularly preferably in the range from -20°C to 150°C. The shell of the inventive emulsion polymer, which is preferably obtainable by polymerization of the inventive CH-acidic methacrylic esters of the formula (I), may preferably have a glass transition temperature in the range from -30°C to 70°C, particularly preferably in the rage from -20°C to 40°C and most particularly preferably in the range from 0°C to 25°C. According to a particular aspect of the present invention, the glass transition temperature of the core may be greater than the glass transition temperature of the shell. Expediently, the glass transition temperature of the core may be at least 10°C, preferably at least 20°C, above the glass transition temperature of the shell.

The copolymers obtainable by polymerization of the inventive CH-acidic methacrylic esters of the formula (I) can be isolated. According to a particular configuration of the present invention, the dispersions obtainable by emulsion polymerization may be used as such as coating composition.

Accordingly, aqueous dispersions are a further subject of the present invention. The aqueous dispersions preferably have a solids content in the range from 10 to 70 wt%, particularly preferably 20 to 60 wt%. Expediently, the dispersion may have a dynamic viscosity in the range from 0.1 to 180 mPas, preferably 1 to 80 mPas and most particularly preferably 10 to 50 mPas, measured to DIN EN ISO 2555 at 25°C (Brookfield).

Furthermore, the inventive aqueous dispersions can be provided in known ways with additives or further components, in order to adapt the properties of the coating composition to specific requirements. These additives especially include drying aids, what are referred to as siccatives, flow improvers, pigments and dyes.

Preferably, the inventive coating compositions have a minimum film-forming temperature of at most 50°C, particularly preferably at most 35°C and most particularly preferably at most 25°C, which can be measured to DIN ISO 2115.

The aqueous dispersions of the present invention can especially be used as coating composition or as additive. This includes, especially, paints, impregnating agents, adhesives and/or primers. The aqueous dispersions can particularly preferably serve for the preparation of paints or impregnating agents for applications on wood and/or metal.

A further aspect of the present invention relates to a coating which is obtainable by applying the inventive aqueous dispersion to an article to be coated and subsequently curing and drying it. In the drying and curing there are generally crosslinking reactions with further components of the aqueous dispersions, and so the inventive coating comprises a crosslinked copolymer. The type of the crosslinking reactions depends on the composition of the aqueous dispersion.

If the inventive aqueous dispersion contains a ketone or an aldehyde, postcrosslinking will occur by a condensation reaction with a ketone or aldehyde when curing the inventive copolymer.

In a further embodiment, the inventive copolymer can also be postcrosslinked by a Michael addition onto an unsaturated bond.

In coating systems containing isocyanate, it is also possible for the inventive copolymer to be postcrosslinked by an addition onto an isocyanate.

The coatings obtainable from the inventive coating compositions exhibit high solvent resistance, with especially only small fractions being dissolved from the coating by solvents. Preferred coatings especially exhibit high resistance to methyl isobutyl ketone (MIBK). Thus, the weight loss after a treatment with MIBK is preferably at most 50 wt%, preferably at most 35 wt%. The absorption of MIBK is preferably at most 300 wt%, particularly preferably at most 250 wt%, based on the weight of the coating used. These values are measured at a temperature of approximately 25°C and a contact time of at least 4 hours, with measurement being carried out on a completely dried coating. In this case, the drying occurs in the presence of oxygen, for example air, in order to enable crosslinking.

The coatings obtained from the inventive coating compositions exhibit high mechanical strength. The pendulum hardness is preferably at least 15 s, preferably at least 25 s, measured to DIN ISO 1522.

The inventive dispersions may also comprise further constituents as well as the emulsions polymers.

The present invention is intended to be described in more detail below using the examples and comparative examples, without this giving rise to any restriction.

## EXAMPLES:

### Analysis

Gas chromatography (GC)
Instrument: 7820A from Agilent Technologies
Column: DB5, 30 m, ø 0.250 mm, 0.25 μm film

### Temperature programme:

Injection at 60°C, then hold for 2 min. Subsequently heat to 240°C at 20°C/min and after reaching that temperature, hold at 240°C for 8 min.

### Example 1: Preparation of N-(2-ethylamino)-2-cyanoacetamide

600 g (10.0 mol) of ethylenediamine are initially charged in a 2 l four-necked round-bottomed flask with sabre stirrer, stirrer motor, thermometer and a 500 ml addition funnel. 248 g (2.5 mol) of methylcyanoacetate are metered in thereto dropwise within 60 minutes, such that the reaction temperature does not exceed 30°C. During this time, the four-necked round-bottomed flask is cooled in an ice-water bath. In the course of the addition of methylcyanoacetate, the reaction mixture becomes increasingly pink coloured, and then lilac. To complete the reaction, the reaction mixture is stirred for a further 90 minutes at room temperature.

Subsequently, the excess ethylenediamine is removed under reduced pressure. For this purpose, the reaction mixture is heated to 100°C (oil bath temperature) and the volatile constituents are distilled off over a period of 2 hours at a pressure of up to 5 mbar.

The product is obtained as a dark, glass-like solid with a purity of 97.9 area% (determined using GC-RV). The product yield is 309 g (95%).

### Comparative Example 1: Preparation of N,N'-ethylenebismethacrylamide

A 40% aqueous solution of ethylenediamine (25.5 g, 0.17 mol) is initially charged in a 250 ml four-necked round-bottomed flask with sabre stirrer, stirrer motor, thermometer and a 100 ml addition funnel. 26 g (0.17 mol) of methacrylic anhydride are metered in thereto dropwise within 60 minutes, such that the reaction temperature does not exceed 30°C. During this time, the four-necked round-bottomed flask is cooled in an ice-water bath. In the course of the addition of methacrylic anhydride, a white solid is formed.

The white solid is separated off by filtration, and dried. It is N,N'-ethylenebismethacrylamide with a purity of

74.8 area% (determined using GC-RV). The product yield is 20 g (60%).

**Example 2: Preparation of N-(2-cyanoethylamidoethyl)methacrylamide**

A mixture of 147 g (1.2 mol) of N-(2-ethylamino)-2-cyanoacetamide and 600 g (6.0 mol) of methylmethacrylate is initially charged in a 1 l four-necked round-bottomed flask with air inlet, sabre stirrer, stirrer motor, and a 50 cm-long 29 mm-thick mirrored column with random packing, filled with 6x6 Raschig rings. 7 mg (10 ppm) of 4-hydroxy-2,2,6,6-tetramethylpiperidinooxyl and 0.15 g (200 ppm) of hydroquinone monomethyl ether are added thereto, followed by 7.4 g of a mixture comprising 65.6 wt% of dioctyltin oxide and 34.4 wt% of tetraisopropyl titanate.

The reaction mixture is heated under reflux, with the methanol forming being distilled off as an azeotrope via the column with random packing. After approximately 3.5 hours, the conversion, determined by GC, is 58%.

**Example 3: Preparation of N-(2-cyanoethylamidoethyl)methacrylamide**

312 g (2.4 mol) of N-(2-ethylamino)-2-cyanoacetamide from example 1 are dissolved in 468 g of water in a 2 l three-necked round-bottomed flask with sabre stirrer, stirrer motor, thermometer and a 500 ml addition funnel. 370 g (2.4 mol) of methacrylic anhydride are slowly added dropwise thereto, with a light brown precipitate being formed. The reaction mixture is then stirred for a further 1.5 hours at 80°C.

The resulting clear, dark red reaction mixture has low boilers removed therefrom under reduced pressure, is concentrated down to 646 g, and has 400 g of isopropanol added thereto. This leads to the formation of a precipitate which is separated off by filtration.

The product is obtained as a brown, crystalline solid with a purity of 95.0 area% (determined using GC-RV). The product yield is 346 g (73.9%).

The solubility of the product in water was determined as follows:

The product was stirred with water for three days at room temperature and the composition of the aqueous phase obtained in this way was determined using HPLC. The aqueous phase contained 6.7 wt% of the product.

A further sample of the product was stirred with water for three hours at 80°C and cooled back to room temperature. The composition of the aqueous phase obtained in this way was subsequently determined using HPLC. The aqueous phase contained more than 10 wt% of the product.

The product has a high water-solubility. Therefore, it can be used in emulsion polymerization even in the absence of organic solvents.

**Example 4: Preparation of N-(2-butylamino)-2-cyanoacetamide**

353 g (4.0 mol) of 1,4-diaminobutane are melted at approximately 30°C in a 1 l four-necked round-bottomed flask with sabre stirrer, stirrer motor, thermometer and a 250 ml addition funnel. 99 g (1.0 mol) of methylcyanoacetate are metered in dropwise thereto within 30 minutes, such that the reaction temperature remains at approximately 30°C to 40°C. During this time, the four-necked round-bottomed flask is cooled in an ice-water bath. In the course of the addition of methylcyanoacetate, the reaction mixture becomes increasingly intensely yellow coloured. To complete the reaction, the reaction mixture is stirred for a further 90 minutes at room temperature, with the reaction mixture becoming red coloured.

Subsequently, excess 1,4-diaminobutane is removed under reduced pressure. For this purpose, the reaction mixture is heated to 100°C (oil bath temperature) and the volatile constituents are distilled off over a period of 2.5 hours at a pressure of up to 2 mbar.

The product is obtained as a dark, glass-like solid with a purity of 89.1 area% (determined using GC-RV). The product yield is 146 g (84%).

**Example 5: Preparation of N-(2-cyanoethylamidobutyl)methacrylamide**

360 g (0.93 mol) of N-(2-butylamino)-2-cyanoacetamide from example 3 are dissolved in 540 g of water in a 1 l four-necked round-bottomed flask with sabre stirrer, stirrer motor, thermometer and a 500 ml addition funnel and cooled to 0°C in an ice-water bath. 143 g (0.93 mol) of methacrylic anhydride, dissolved in 300 ml of methanol, are slowly added thereto dropwise. Subsequently, the reaction mixture is stirred overnight at room temperature, with the reaction mixture becoming green coloured.

The reaction mixture is concentrated under reduced pressure at 80°C and 35 mbar to 263 g. The residue is dissolved in 160 g of isopropanol and the resulting solution is stored at room temperature. This leads to the formation of a precipitate which is separated off by filtration.

The product is obtained as a yellow crystalline solid. The purity is approximately 94.0 area% (determined using

GC-RV).

The solubility of the product in water was determined as follows:
The product was stirred with water for two days at room temperature and the composition of the aqueous phase obtained in this way was determined using HPLC. The aqueous phase contained 6.2 wt% of the product.

A further sample of the product was stirred with water for one hour at 60°C and cooled back to room temperature. The composition of the aqueous phase obtained in this way was subsequently determined using HPLC. The aqueous phase contained 8.1 wt% of the product.

The product has a high water-solubility. Therefore, it can be used in emulsion polymerization even in the absence of organic solvents.

**Comparative Example 2: Preparation of N-(2-hexylamino)-2-cyanoacetamide**

465 g (4.0 mol) of 1,6-diaminohexane are melted at approximately 41°C in a 1 l four-necked round-bottomed flask with sabre stirrer, stirrer motor, thermometer and a 250 ml addition funnel. 99 g (1.0 mol) of methylcyanoacetate are metered in dropwise thereto within 30 minutes, such that the reaction temperature remains at approximately 50°C to 75°C. In the course of the addition of methylcyanoacetate, the reaction mixture becomes increasingly intensely yellow coloured. To complete the reaction, the reaction mixture is stirred for a further 90 minutes at approximately 50°C to 75°C, with the reaction mixture becoming red coloured.

Subsequently, the excess 1,6-diaminohexane is removed under reduced pressure. For this purpose, the reaction mixture is heated to 120°C (oil bath temperature) and the volatile constituents are distilled off over a period of 4 hours at a pressure of up to 2 mbar.

The product is obtained as a dark, glass-like solid with a purity of approximately 100 area% (determined using GC-RV). The product yield is 172 g (94%).

**Comparative Example 3: Preparation of N-(2-cyanoethylamidohexyl)methacrylamide**

31 g (0.2 mol) of methacrylic anhydride and 150 g of water are initially charged in a 1 l four-necked round-bottomed flask with sabre stirrer, stirrer motor, thermometer and a 500 ml addition funnel and cooled to 0°C in an ice-water bath.

360 g (0.93 mol) of N-(2-hexylamino)-2-cyanoacetamide from comparative example 2 are dissolved in 3240 g of methanol at 60°C and cooled to room temperature. This solution is added over a period of 30 minutes via the addition funnel to methacrylic anhydride. The reaction temperature is kept below 20°C. The reaction mixture is then stirred for a further 3 hours at room temperature.

The product formed is detected in the reaction mixture using GC-RV and can be isolated by crystallization from isopropanol.

The solubility of the product in water was determined as follows:
The product was stirred with water for three days at room temperature and the composition of the aqueous phase obtained in this way was determined using HPLC. The product is virtually insoluble in water.

A further sample of the product was stirred with water for three hours at 80°C and cooled to room temperature. The composition of the aqueous phase obtained in this way was subsequently determined using HPLC. The product is virtually insoluble in water; only impurities from the product are dissolved and detected.

The product has a very low water-solubility. Therefore, it cannot be used in emulsion polymerization in the absence of organic solvents.

## Claims

1. Compound of the general formula (I):

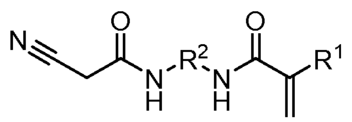

(I)

wherein $R^1$ is selected from a hydrogen atom and a methyl group and
$R^2$ is an optionally substituted alkylene group of the composition

$C_nH_m$,

with

n = 2 - 4
m = 2 - 8.

2. Compound according to Claim 1, wherein the alkylene group $R^2$ is one of the following groups: 1,2-ethylene group, 1,3-propylene group, 1,2-propylene group, 1,4-butylene group, 1,3-(2-hydroxy)propylene group, 1,3-(2,2-dimethyl)propylene group.

3. Compound according to at least one of Claims 1 to 2, wherein $R^1$ is a methyl group.

4. Copolymer obtainable by polymerization of a monomer mixture comprising at least 0.5 wt%, preferably at least 1.0 wt% of the compound according to at least one of Claims 1 to 3, based on the weight of the monomer mixture.

5. Copolymer according to Claim 4, **characterized in that** the monomer mixture comprises 0.5 to 90 wt% of the compound according to at least one of Claims 1 to 3,
10 to 95 wt% of at least one monomer comprising ester groups,
0 to 10 wt% of at least one monomer with an acid group,
0 to 50 wt% of at least one styrene monomer, based on the weight of the copolymer.

6. Aqueous dispersion comprising 0.1 to 90 wt%, preferably 20 to 80 wt%, particularly preferably 30 to 60 wt% of the copolymer according to Claim 4 or 5, based on the weight of the aqueous dispersion.

7. Use of the aqueous dispersion according to Claim 6 as a coating composition, especially as emulsion paint, clearcoat, facade application, protective paint, ship coating or metal coating.

8. Coating, obtainable by applying the aqueous dispersion according to Claim 6 to an article to be coated and subsequently drying and curing it.

9. Coating according to Claim 8, wherein the aqueous dispersion according to Claim 6 contains an aldehyde or a ketone which enters into a condensation reaction with the copolymer according to Claim 4 or 5 during the curing.

10. Coating according to Claim 8, wherein the aqueous dispersion according to Claim 6 contains at least one aldehyde or a ketone which enters into a condensation reaction with the copolymer according to Claim 4 or 5 during the curing.

11. Coating according to Claim 8 wherein the aqueous dispersion according to Claim 6 contains an isocyanate which enters into addition reactions with the copolymer according to Claim 4 or 5 during the curing.

**Patentansprüche**

1. Verbindung der allgemeinen Formel (I):

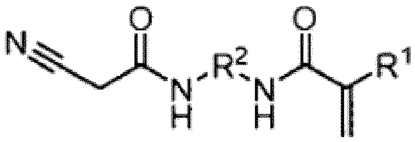

(I)

,

wobei $R^1$ aus einem Wasserstoffatom und einer Methylgruppe ausgewählt ist und $R^2$ für eine gegebenenfalls substituierte Alkylengruppe der Zusammensetzung

$C_nH_m$

steht, mit

n = 2-4
m = 2-8.

2. Verbindung nach Anspruch 1, wobei es sich bei der Alkylengruppe $R^2$ um eine der folgenden Gruppen handelt: 1,2-Ethylengruppe, 1,3-Propylengruppe, 1,2-Propylengruppe, 1,4-Butylengruppe, 1,3-(2-Hydroxy)propylengruppe, 1,3-(2,2-Dimethyl)propylengruppe.

3. Verbindung nach einem der Ansprüche 1 bis 2, wobei $R^1$ für eine Methylgruppe steht.

4. Copolymer, das durch Polymerisation einer Monomermischung erhältlich ist, die, bezogen auf das Gewicht der Monomermischung, mindestens 0,5 Gew.-%, vorzugsweise mindestens 1,0 Gew.-%, der Verbindung nach einem der Ansprüche 1 bis 3 umfasst.

5. Copolymer nach Anspruch 4, **dadurch gekennzeichnet, dass** die Monomermischung, bezogen auf das Gewicht des Copolymers,
0,5 bis 90 Gew.-% der Verbindung nach einem der Ansprüche 1 bis 3,
10 bis 95 Gew.-% mindestens eines Estergruppen umfassenden Monomers,
0 bis 10 Gew.-% mindestens eines Monomers mit einer Säuregruppe,
0 bis 50 Gew.-% mindestens eines Styrolmonomers, umfasst.

6. Wässrige Dispersion, die, bezogen auf das Gewicht der wässrigen Dispersion, 0,1 bis 90 Gew.-%, vorzugsweise 20 bis 80 Gew.-%, besonders bevorzugt 30 bis 60 Gew.-%, des Copolymers nach einem der Ansprüche 4 oder 5 umfasst.

7. Verwendung der wässrigen Dispersion nach Anspruch 6 als Lackzusammensetzung, insbesondere als Dispersionsanstrich, Klarlack, Fassadenanstrich, Schutzanstrich, Schiffslack oder Metalllack.

8. Lack, erhältlich durch Auftragen der wässrigen Dispersion nach Anspruch 6 auf einen zu lackierenden Gegenstand und anschließende Trocknung und Härtung von diesem.

9. Lack nach Anspruch 8, wobei die wässrige Dispersion nach Anspruch 6 einen Aldehyd oder ein Keton enthält, der bzw. das mit dem Copolymer nach Anspruch 4 oder 5 während des Härtens eine Kondensationsreaktion eingeht.

10. Lack nach Anspruch 8, wobei die wässrige Dispersion nach Anspruch 6 mindestens einen Aldehyd oder ein Keton enthält, der bzw. das mit dem Copolymer nach Anspruch 4 oder 5 während des Härtens eine Kondensationsreaktion eingeht.

11. Lack nach Anspruch 8, wobei die wässrige Dispersion nach Anspruch 6 ein Isocyanat enthält, das mit dem Copolymer nach Anspruch 4 oder 5 während des Härtens eine Additionsreaktion eingeht.

**Revendications**

1. Composé de formule générale (I) :

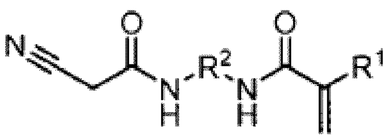

(I)

$R^1$ étant choisi parmi un atome d'hydrogène et un groupe méthyle et $R^2$ étant un groupe alkylène éventuellement substitué de composition

$C_nH_m$,

avec

n = 2 à 4
m = 2 à 8.

**2.** Composé selon la revendication 1, le groupe alkylène $R^2$ étant l'un des groupes suivants : groupe 1,2-éthylène, groupe 1,3-propylène, groupe 1,2-propylène, groupe 1,4-butylène, groupe 1,3-(2-hydroxy)propylène, groupe 1,3-(2,2-diméthyl)propylène.

**3.** Composé selon au moins l'une des revendications 1 et 2, $R^1$ étant un groupe méthyle.

**4.** Copolymère pouvant être obtenu par polymérisation d'un mélange de monomères comprenant au moins 0,5 % en poids, préférablement au moins 1,0 % en poids du composé selon au moins l'une des revendications 1 à 3, sur la base du poids du mélange de monomères.

**5.** Copolymère selon la revendication 4, **caractérisé en ce que** le mélange de monomère comprend
0,5 à 90 % en poids du composé selon au moins l'une des revendications 1 à 3,
10 à 95 % en poids d'au moins un monomère comprenant des groupes ester,
0 à 10 % en poids d'au moins un monomère comportant un groupe de type acide,
0 à 50 % en poids d'au moins un monomère de type styrène, sur la base du poids du copolymère.

**6.** Dispersion aqueuse comprenant 0,1 à 90 % en poids, préférablement 20 à 80 % en poids, particulièrement préférablement 30 à 60 % en poids du copolymère selon la revendication 4 ou 5, sur la base du poids de la dispersion aqueuse.

**7.** Utilisation de la dispersion aqueuse selon la revendication 6 en tant que composition de revêtement, notamment en tant que peinture à émulsion, revêtement transparent, application de façade, peinture protectrice, revêtement de bateau ou revêtement de métal.

**8.** Revêtement, qui peut être obtenu par l'application de la dispersion aqueuse selon la revendication 6 à un article devant être revêtu et subséquemment par séchage et durcissement de celle-ci.

**9.** Revêtement selon la revendication 8, la dispersion aqueuse selon la revendication 6 contenant un aldéhyde ou une cétone qui entre dans une réaction de condensation avec le copolymère selon la revendication 4 ou 5 pendant le durcissement.

**10.** Revêtement selon la revendication 8, la dispersion aqueuse selon la revendication 6 contenant au moins un aldéhyde ou une cétone qui entre dans une réaction de condensation avec le copolymère selon la revendication 4 ou 5 pendant le durcissement.

**11.** Revêtement selon la revendication 8, la dispersion aqueuse selon la revendication 6 contenant un isocyanate qui entre dans des réactions d'addition avec le copolymère selon la revendication 4 ou 5 pendant le durcissement.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0016518 A
- DE 4237030
- WO 2009146995 A1
- EP 2246403 A


### Non-patent literature cited in the description


- Ullmann's Encyclopedia of Industrial Chemistry. Wiley-VCH, 2003
- Römpp Chemielexikon
- Römpp-Lexikon Chemie. 1996
- **W. GÖSELE, CHR. ALT.** Ullmann's Encyclopedia of Industrial Chemistry. Wiley-VCH publishing, 2003, vol. 13, 731, , 746
- Ullmann's Encyclopedia of Industrial Chemistry
- Chemistry and Technology of Emulsion Polymerisation. Blackwell Publishing, 2005
- **J. O'DONNELL ; E.W. KALER.** *Macromolecular Rapid Communications,* 2007, vol. 28 (14), 1445-1454
- **H. RAUCH-PUNTIGAM ; TH. VÖLKER.** Acryl- und Methacrylverbindungen" [acrylic and methacrylic compounds. Springer, 1967
- **KIRK-OTHMER.** Encyclopedia of Chemical Technology. J. Wiley, 1978, vol. 1, 386ff
- **FOX T. G.** *Bull. Am. Physics Soc.,* 1956, vol. 1 (3), 123
- Polymer Handbook. J. Wiley & Sons, 1975